# EUROPEAN PATENT SPECIFICATION

(11) **EP 3 898 577 B1**
(45) Date of publication and mention of the grant of the patent: **08.02.2023**
(21) Application number: 19820776.3
(22) Date of filing: 17.12.2019
(51) Int. Cl.: C07C 231/02, C07C 233/18, C07C 233/20, C11D 1/52, C11D 1/645

(54) **GLUCAMIDE-BASED SURFACTANTS**
TENSIDE AUF GLUCAMIDBASIS
TENSIOACTIFS À BASE DE GLUCAMIDE

(30) Priority: 21.12.2018 EP 18215374
(43) Date of publication of application: 27.10.2021
(73) Proprietor: Clariant International Ltd, 4132 Muttenz (CH)
(72) Inventor: PURKAYASTHA, Nirupam, 61169 Friedberg (DE); LEINWEBER, Dirk, 65779 Kelkheim (DE); KUPPERT, Dirk, Roland, 60486 Frankfurt am Main (DE)
(74) Representative: Paczkowski, Marcus
(86) International application number: PCT/EP2019/085614
(87) International publication number: WO 2020/127237

(56) References cited:
- EP-A1- 1 072 615
- WO-A1-2018/099768

## Description

### FIELD OF THE INVENTION

The present invention generally relates to surfactants. In particular, it relates to glucamide-based surfactants, as well as processes for synthesizing them and the resultant reaction products, and solutions and formulations comprising such surfactants. The glucamide-based surfactants can be employed in a wide range of applications.

### BACKGROUND OF THE INVENTION

Surfactants (short for `surface-active agents') due to their amphiphilic character, which gives them an affinity for both hydrophilic and lipophilic environments play an important role as cleaning, wetting, dispersing, emulsifying, foaming and antifoaming agents in many practical applications and products, including: detergents, personal and home care formulations, agrochemical formulations, industrial and oil field applications, etc.

Many materials employed in the production of surfactants are traditionally derived from crude oil. Environmental, economic and sustainability questions are restricting the use of products derived from this limited resource: synthetic surfactants, for example, have been blamed for environmental incidents, particularly vis-à-vis aquatic problems in rivers and lakes. Therefore, there is a desire to identify more sustainable and biodegradable, yet gentle and effective materials. Indeed, consumers are very interested in "natural" products including products with a high percentage of "natural" compounds and/or compounds that are derived from renewable materials. Consumers perceive compounds derived from natural materials to be gentler and more environmentally friendly. Recent industrial developments in "bio-based" chemicals are summarised, for example, in de Jong et al, "Product developments in the bio-based chemicals arena", Biofuels, Bioprod. Bioref. 6:606-624 (2012). In relation to surfactants in particular, discussed in Global Business Briefing, October 2011 / Europe the "Market opportunities beckon for bio-surfactants" (see https://chemicalwatch.com/8727/market-opportunities-beckon-for-bio-surfactants).

Compounds derived from natural materials have various other benefits, including increased biodegradability and also more sustainable availability because they are not based on a limited resources. Compounds derived from plant-based resources are particularly useful since the source compound can simply be regrown. Consumers are also particularly comfortable with using compounds derived from well-known plants, especially those that are considered staple products.

Consequently, there is a need for surfactants that derived from natural materials and also have an improved safety profile and reduced health concerns. Such new surfactants must also provide excellent performance, particularly vis-à-vis foamability.

US-A 5,510,049 describes laundry or toilet bars comprising one or more surface active agents, such as anionic surfactants, soaps, and synthetic detergents prepared using N-alkylether N-polyhydroxy fatty acid amide. The hydrocarbon moiety of the fatty acid is preferably a C₉ to C₂₁ hydrocarbyl straight chain substituted or unsubstituted alkyl and alkenyl, e.g. 12-hydroxyoleic acid. Palm oil chain-length fatty acid amides of N-(3-methoxypropyl) glucamine and N-(2-methoxyethyl) glucamine are examples of the glucamide surfactant used in such bars.

In WO 2013/178668 A2 a water based surfactant solution is described, which is suitable for cosmetic application comprising a mixture of (a1) 5 to 20 wt.-% of N-methyl-N-oleylglucamide, (a2) 50 to 93 wt.-% of N-methyl-N-C12-C14-acylglucamides and (a3) 0 to 30 wt.-% of other N-methyl-N-acylglucamides.

WO 2018/099768 A1 describes a composition in the form of a gel or a paste comprising a blend of different N-hydrocarbon substituted-N-acylglucamides a1) to a4) of the formula below 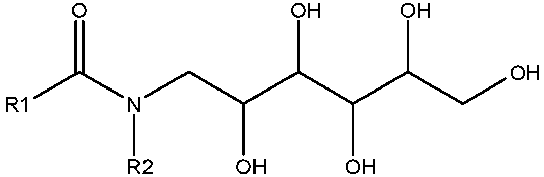 wherein R1 is preferably a linear C₇-C₁₇-alkyl group or mono- or polyunsaturated C₇-C₁₇-alkenyl group.

The examples show blends comprising N-methyl-N-C₈/C₁₀-acylglucamide a1), N-methyl-C₁₂/C₁₄-acylglucamide a2), N-methyl-N-oleylglucamide a3) and a non-specified N-methyl-N-acylglucamide a4).

However, the compounds described in the prior art are often based on palm oil. While palm oil is the most efficient source of vegetable oil, its rapid expansion threatens some of the planet's most important and sensitive habitats. Vast expanses of rainforests are cut own for palm oil production, endangered species such as the orangutan are driven from their habitat, and land-use rights are being ignored. Thus, it is desirable to use renewable raw materials alternative to palm oil based.

It is an object of the present invention to offer novel glucamide-based surfactants based on renewable raw materials alternative to palm oil based.

Another objective of the present invention is to offer glucamide-based surfactants having better or at least equivalent properties, in particular with respect to surface tension, critical micelle concentration (CMC), water solubility and foam behavior than the compounds of the prior art.

Additionally an objective of the present invention is to provide bio-based surfactants based on renewable raw materials. It is another object of the instant invention to provide a composition that can be used for various applications, and that is biodegradable.

Surprisingly, it has now been found that the above objective can be solved by novel N-acylglucamides and further by an aqueous surfactant solution comprising said N-acylglucamides according to the claims.

### SUMMARY OF THE INVENTION

In a first aspect, the present invention relates to N-acylglucamide compounds of the formula (I) 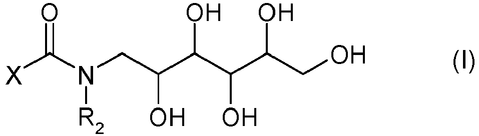 where
- R₂: is a linear or branched alkyl group or an aryl group, preferably a linear alkyl group comprising 1 to 6 carbon atoms or a phenyl group, more preferred a methyl, ethyl, propyl or butyl group and most preferred a methyl group;
- X: is a moiety R₁, which is a linear or branched, preferably a linear, alkyl group comprising 8 to 18, preferably 13 to 17, in particular preferably 13 to 15, carbon atoms, which is substituted by one hydroxy group preferably in beta- or omega-position,
most preferred an alkyl group with 13 carbon atoms substituted by a hydroxy group in beta-position, or an alkyl group with 15 carbon atoms substituted by a hydroxy group in omega-position; or
- X: is a moiety R_{1'}, which is a linear or branched, preferably a linear, mono- or polyunsaturated, preferably monounsaturated, alkenyl group comprising 8 to 18, preferably 13 to 17, in particular preferably 13 to 15, carbon atoms, which is substituted by one hydroxy group preferably in beta- or omega-position,
most preferred a monounsaturated alkenyl group with 13 carbon atoms substituted by a hydroxy group in beta-position, or a monounsaturated alkenyl group with 15 carbon atoms substituted by a hydroxy group in omega-position.

According to at least one embodiment the moiety R_{1'} is as defined above but with the proviso that it is not a moiety derived from 12-hydroxyoleic acid.

### DETAILED DESCRIPTION OF THE INVENTION

### Definitions and General

In this document, including in all embodiments of all aspects of the present invention, the following definitions apply unless specifically stated otherwise.

In this document, including in all embodiments of all aspects of the present invention, the following definitions apply unless specifically stated otherwise. All percentages are by weight (w/w) of the total composition. "wt%", "wt.%" or "wt.-%" means percentage by weight. All ratios are weight ratios.

The term "alkenyl group comprising 8 to 18 carbon atoms, which is substituted by one, hydroxy group" as used herein for the moiety R_{1'} of the compound of formula (I) above in particular means that one carbon atom of a carbon single bond of the moiety R_{1'} is substituted by a hydroxy group.

References to 'parts' e.g. a mixture of 1 part X and 3 parts Y, is a ratio by weight. All ranges are inclusive and combinable.

The number of significant digits conveys neither a limitation on the indicated amounts nor on the accuracy of the measurements. All numerical amounts are understood to be modified by the word "about".

All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International.

Herein "min" means "minute" or "minutes". Herein "mol" means mole. Herein "g" following a number means "gram" or "grams". "Ex." means "example".

All amounts as they pertain to listed ingredients are based on the active level ('solids') and do not include carriers or by-products that may be included in commercially available materials.

Herein, "comprising" means that other steps and other ingredients can be in addition. "Comprising" encompasses the terms "consisting of" and "consisting essentially of". The compositions, formulations, uses, and processes of the present invention can comprise, consist of, and consist essentially of the elements and limitations of the invention described herein, as well as any of the additional or optional ingredients, components, steps, or limitations described herein.

Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature.

Where amount ranges are given, these are to be understood as being the total amount of said ingredient in the composition, or where more than one species fall within the scope of the ingredient definition, the total amount of all ingredients fitting that definition, in the composition.

"Independently selected from," means that the referenced groups can be the same, different, or a mixture thereof, unless the context clearly indicates otherwise.

"Molecular weight" or "M.Wt." or "MW" and grammatical equivalents mean the number average molecular weight.

"Water-soluble" refers to any material that is sufficiently soluble in water to form a clear solution to the naked eye at a concentration of 0.1 % by weight of the material in water at 25°C. The term "water-insoluble" refers to any material that is not "water-soluble".

"Substantially free from" or "substantially free of" means less than 1 %, or less than 0.8%, or less than 0.5%, or less than 0.3%, or about 0%, by total weight of the composition.

"Derivatives" includes but is not limited to, amide, ether, ester, amino, carboxyl, acetyl, acid, salt and/or alcohol derivatives of a given compound. In at least one embodiment, "derivatives thereof' means the amide, ether, ester, amino, carboxyl, acetyl, acid, salt and alcohol derivatives.

The details of the invention and its aspects are provided hereinafter.

### First Aspect

In a first aspect, the present invention relates to N-acylglucamide compounds of the formula (I) 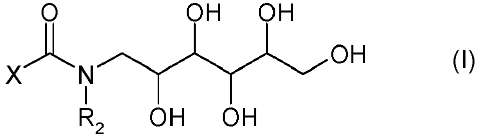 where
- R₂: is a linear or branched alkyl group or an aryl group, preferably a linear alkyl group comprising 1 to 6 carbon atoms or a phenyl group, more preferred a methyl, ethyl, propyl or butyl group and most preferred a methyl group;
- X: is a moiety R₁, which is a linear or branched, preferably a linear, alkyl group comprising 8 to 18, preferably 13 to 17, in particular preferably 13 to 15, carbon atoms, which is substituted by one hydroxy group preferably in beta- or omega-position,
most preferred an alkyl group with 13 carbon atoms substituted by a hydroxy group in beta-position, or an alkyl group with 15 carbon atoms substituted by a hydroxy group in omega-position; or
- X: is a moiety R_{1'}, which is a linear or branched, preferably a linear, mono- or polyunsaturated, preferably monounsaturated, alkenyl group comprising 8 to 18, preferably 13 to 17, in particular preferably 13 to 15, carbon atoms, which is substituted by one hydroxy group preferably in beta- or omega-position,
most preferred a monounsaturated alkenyl group with 13 carbon atoms substituted by a hydroxy group in beta-position, or a monounsaturated alkenyl group with 15 carbon atoms substituted by a hydroxy group in omega-position.

Preferably, in the compound of formula (I) according to the invention as described above,
- R₂: is a linear alkyl group comprising 1 to 6 carbon atoms, preferably a methyl, ethyl, propyl or butyl group and more preferred a methyl group;
- X: is a moiety R₁, which is a linear alkyl group comprising 13 to 17, preferably 13 to 15, carbon atoms, which is substituted by one hydroxy group preferably in beta- or omega-position, more preferred an alkyl group with 13 carbon atoms substituted by a hydroxy group in beta-position, or an alkyl group with 15 carbon atoms substituted by a hydroxy group in omega-position; or
- X: is a moiety R_{1'}, which is a linear, monounsaturated alkenyl group comprising 13 to 17, preferably 13 to 15, carbon atoms, which is substituted by one hydroxy group preferably in beta- or omega-position, more preferred a monounsaturated alkenyl group with 13 carbon atoms substituted by a hydroxy group in beta-position, or a monounsaturated alkenyl group with 15 carbon atoms substituted by a hydroxy group in omega-position.

More preferably, in the compound of formula (I) according to the invention as described above,
- R₂: is methyl, ethyl, or a linear propyl or butyl group, preferably a methyl group;
- X: is a moiety R₁, which is a linear alkyl group comprising 13 to 17, preferably 13 to 15, carbon atoms, which is substituted by one hydroxy group in beta- or omega-position; or
- X: is a moiety R_{1'}, which is a linear, monounsaturated alkenyl group comprising 13 to 17, preferably 13 to 15, carbon atoms, which is substituted by one hydroxy group in beta- or omega-position.

Most preferably, in the compound of formula (I) according to the invention as described above,
- R₂: is a methyl group;
- X: is a moiety R₁, which is a linear alkyl group with 13 carbon atoms substituted by a hydroxy group in beta-position; or
- X: is a moiety R_{1'}, which is a linear, mono-unsaturated alkenyl group with 13 carbon atoms substituted by a hydroxy group in beta-position.

Furthermore, most preferably in the compound of formula (I) according to the invention as described above,
- R₂: is a methyl group;
- X: is a moiety R₁, which is a linear alkyl group with 15 carbon atoms substituted by a hydroxy group in omega-position; or
- X: is a moiety R_{1'}, which is a linear mono-unsaturated alkenyl group with 15 carbon atoms substituted by a hydroxy group in omega-position.

Preferably the compounds of the formula (I) according to the invention as described above wherein X is a moiety R_{1'} are cis-isomers.

A further aspect of the invention are blends consisting of at least one, preferably one, N-acylglucamide compound of the formula (Ia) 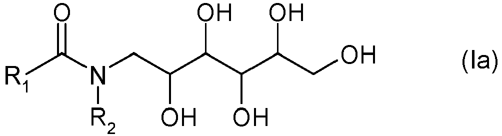 and at least one, preferably one, N-acylglucamide compound of the formula (Ib) 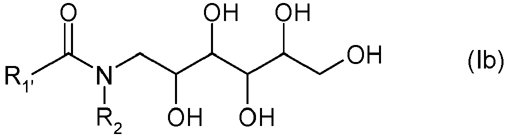 wherein R₂, R₁ and R_{1'} are as defined for the compounds of the formula (I) above.

Preferably the compounds of the formulas (Ia) and (Ib) are identical, except of the extent of unsaturation of the moieties R₁ and R_{1'}.

Furthermore preferred the amount of the compound of the formula (Ib) - based on the blend of the compounds of the formulas (Ia) and (Ib) - is at least 35 wt.-%, preferably at least 40 wt.-%, more preferably at least 65 wt.-%, most preferably at least 70 wt.-%.

"Identical, except of the extent of unsaturation of moieties R₁ and R_{1'}" of the compounds of the formulas (Ia) and (Ib) as defined above means the compounds of the formulas (Ia) and (Ib) have an identical structure (e.g. linear or branched), number of carbon atoms and substitution, but in difference to the unsaturated moiety R_{1'} of the compound of the formula (Ib), the moiety R₁ of the compound of the formula (Ia) is completely saturated.

In particular preferred are afore-mentioned blends of N-acylglucamide compounds of the formulas (Ia) and (Ib), where
- R₂: is a methyl group;
- R₁: is a linear alkyl group with 13 carbon atoms substituted by a hydroxy group in beta-position;
- R_{1'}: is a linear, mono-unsaturated alkenyl group with 13 carbon atoms substituted by a hydroxy group in beta-position; and
the amount of the compound of the formula (Ib) - based on the blend of (Ia) and (Ib) - is at least 40 wt.-%.

Furthermore, in particular preferred are afore-mentioned blends of N-acylglucamide compounds of the formulas (Ia) and (Ib), where
- R₂: is a methyl group;
- R₁: is a linear alkyl group with 15 carbon atoms substituted by a hydroxy group in omega-position;
- R_{1'}: is a linear mono-unsaturated alkenyl group with 15 carbon atoms substituted by a hydroxy group in omega-position; and
the amount of the compound of the formula (Ib) - based on the blend of (Ia) and (Ib) - is at least 70 wt.-%.

The compounds of the formula (I) according to the invention and the inventive blends of N-acylglucamide compounds of the formulas (Ia) and (Ib) may be prepared by methods well-known to the person skilled in the art.

### Second Aspect

In a second aspect, the invention provides a process for synthesizing the compounds of the formula (I) according to the invention or the inventive blends of N-acylglucamide compounds of the formulas (Ia) and (Ib) as described above, wherein the process comprises:
i) Providing fatty acids or fatty acid derivatives such as fatty acid esters or fatty acid halogenides of the formula XCOR³, wherein X is as described in formula (I) above and R³ is hydroxy, a linear or branched C₁-C₄-alkoxy group or halogen (F, Cl, Br, J);
ii) Amidation of the fatty acid or fatty acid derivative of the formula XCOR³, with an N-alkyl- or aryl-glucamine, preferably N-alkyl-glucamine, more preferably N-methyl-glucamine, in a polar protic solvent in presence of a base catalyst.

The fatty acid derivatives of the formula XCOR³ used in said process are preferably fatty acid esters, more preferably fatty acid esters of the formula XCOO(C₁-C₄-alkyl), most preferably fatty acid methyl esters of formula XCOOCH₃, wherein X is as described in formula (I) above and R³ is an C₁-C₄-alkoxy group, in particular methoxy group.

Preferably the fatty acids or fatty acid derivatives of the formula XCOR³, in particular fatty acid esters of the formula XCOO(C₁-C₄-alkyl), are obtained by living organisms from simple carbon renewable raw materials (feedstocks). A simple carbon source that is derived from a renewable feedstock, including but not limited to, carbohydrates from corn, cane, natural gas, or lignocellulosic biomass; waste products such as municipal solid waste, glycerol, flu-gas, syn-gas, carbon dioxide; or the carbon streams resulting from the reformation of organic materials such as biomass, natural gas, or other carbonaceous materials.

Carbohydrates, in particular sugars, are preferably used as renewable feedstock.

Preferably the fatty acids or fatty acid derivatives of the formula XCOR³, in particular fatty acid esters of the formula XCOO(C₁-C₄-alkyl), provided in step i) are obtained by living organisms from said simple carbon renewable raw materials, preferably carbohydrates, in particular sugars, in a host cell that contains one or more naturally-occurring mutations that result in an increased level of fatty acids and/or fatty acid derivatives in the host cell. In some instances, the host cell is genetically engineered to increase the level of fatty acids and/or fatty acid derivatives in the host cell relative to a corresponding wild-type host cell. For example, the host cell can be genetically engineered to express a reduced level of a fatty acid degradation enzyme. Such as an acyl-CoA synthase relative to a corresponding wild-type host cell. In one embodiment, the level of expression of one or more genes (e.g., an acyl-CoA synthase gene) is attenuated or functionally deleted by genetically engineering a "knock out host cell". Finally the fatty acids or fatty acid derivatives of the above-formula are separated from the respective growth medium.

Such processes are described in WO 2007/136762 A2, US 2011/0072714 A1 and WO 2016/011430 A1.

Amidation processes of fatty acid esters and N-alkyl polyhydroxy amines are known and described in detail in WO 92/06073 A1.

Preferred fatty acid methyl esters such as Methyl-3-hydroxytetradecanoate (partially unsaturated or completely saturated) and Methyl-16-hydroxyhexadecanoate (partially unsaturated or completely saturated) used in said process according to the invention , are obtained by living organisms from simple carbon renewable raw materials.

### Third Aspect

A third aspect relates to an aqueous surfactant solution comprising one or more, preferably one, compound(s) of the formula (I) according to the invention, or the blend of N-acylglucamide compounds of the formulas (Ia) and (Ib) according to the invention as described above.

Preferably the aqueous surfactant solution according to the invention comprises (or consists of):
a) 0.1 to 10 wt.-% of one or more, preferably one, compound(s) of the formula (I), or of the blend of N-acylglucamide compounds of the formulas (Ia) and (Ib),
b) 50 to 99.9 wt.-% of water,
c) 0 to 49.9 wt.-% of one or more further additives,
wherein a), b) and c) sum up to 100 wt.-%.

More preferably the aqueous surfactant solution according to the invention comprises (or consists of):
a) 0.1 to 5 wt.-% of one or more, preferably one, compound(s) of the formula (I), or of the blend of N-acylglucamide compounds of the formulas (Ia) and (Ib),
b) 75 to 99.9 wt.-% of water,
c) 0 to 20 wt.-% of one or more additives,
wherein a), b) and c) sum up to 100 wt.-%.

In a preferred embodiment of the invention, the additives c) are one or more substances selected from the group consisting of non-ionic surfactants other than the glucamides of formula (I), cationic surfactants, anionic surfactants, amphoteric surfactants, auxiliaries which are customary and specific in each case, for example additives for dissolution time control, additives for performance improvement (e.g. soil release polymers; dye fixatives; dye transfer inhibitors; bleach systems; biocides or antimicrobial components; or builders) or washing assistants (e.g. enzymes; enzyme stabilizers; preservatives; foam enhancers; foam inhibitors; corrosion inhibitors; optical brighteners; UV absorbers; alkalis; hydrotropic compounds; antioxidants; solvents; salts or extenders; dispersants; graying inhibitors; softeners; antistats; fragrances or perfumes); dyes; pigments; colorants or pearlizing agents.

Nonionic surfactants other than glucamides of formula (I) that may be contained in the aqueous surfactant solution according to the invention are further N-acylglucamides, alkanolamides, alkyldimethyl-amineoxides, di-alkyl-methylamineoxides, alkylamidopropyl-amine oxides, fatty acid-N-methylglucamides different from those of component a) of the inventive aqueous surfactant solution, alkylpolyglucosides, oxalkylated fatty acids, alkoxylated alcohols (alkyl ethers, alkyl polyethylene glycol ethers, fatty alcohol polyglycol ethers), (EO/PO) ethoxylated/propoxylated block copolymers, oxalkylated fatty acid esters and oxalkylated alkylamines. The alkyl and fatty acid groups of these compounds, which also may be fully or partially replaced by the corresponding unsaturated groups, may contain 8 to 22 carbon atoms and may be linear or branched. Oxalkylated means products that contain preferably 1 to 20 units of ethylene oxide or propyleneoxide or mixtures thereof.

Preferably additives c) do not contain any further N-acylglucamide.

The amount of the one or more other nonionic surfactants which can be added to the aqueous surfactant solution according to the invention preferably is of from 0.1 to 30 % by weight and more preferably of from 0.5 to 25 % by weight.

The aqueous surfactant solution according to the invention may contain one or more alcohol ethoxylates of formula (II) as non-ionic surfactants

R⁴-O-OCH₂CH₂O)ₙ-H (II)

wherein
- R⁴: is a linear or branched, preferably a linear, saturated alkyl group comprising 12 to 22, preferably 12 to 20 and more preferably 16 to 18, carbon atoms or a linear or branched, preferably a linear, mono- or polyunsaturated alkenyl group comprising 12 to 22, preferably 12 to 20 and more preferably 16 to 18, carbon atoms,
whereby the alcohol R⁴-OH underlying the respective alcohol ethoxylate of the formula (II) possesses a iodine number of from 30 to 135 g(J₂)/100 g alcohol R⁴-OH and preferably of from 36 to 120 g(J₂)/100g alcohol R⁴-OH, and
- n: on a molar average, is a number of from 4 to 50 and preferably of from 5 to 25.

Preferred aqueous surfactant solutions according to the invention do not contain any alcohol ethoxylates, especially no alcohol ethoxylates of formula (II).

As cationic surfactants there may be used quaternary ammonium compounds such as alkyldimethyl-hydroxyethyl-ammonium. Instead of alkyl these ammonium compounds may also have alkenyl groups or mixtures of both. The alkyl as well as the alkenyl groups may contain 8 to 22 carbon atoms. They may be linear or branched. Preferred ammonium compounds are C₈-C₂₂-alkyl- or alkenyl-dimethyl-hydroxyethyl-ammonium compounds. Particularly preferred ammonium compounds are C₁₂/C₁₄-alkyl dimethyl hydroxyethyl ammonium compounds. All mentioned ammonium compounds may contain any kind of anion, the preferred ones are chloride, bromine, acetate, lactate, sulfate or methosulfate. A very preferred ammonium compound is C₁₂/C₁₄-alkyl dimethyl hydroxyethyl ammonium chloride.

Anionic surfactants may be selected from the group consisting of alkyl sulfates, alkyl ether sulfates, alkyl aryl sulfonates such as a linear alkylbenzene sulfonate (LAS), fatty acid soaps, methyl ester sulfonates, paraffin sulfonates, olefin sulfonates, alcohol ethoxysulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acyl amino acids such as, for example, acyl lactylates, acyl tartrates, acyl glutamates and alkyl oligoglucoside sulfates.

Furthermore, the aqueous surfactant solution according to the invention may contain 0.1 to 12 % by weight and preferably 0.2 to 10 % by weight of amphoteric surfactants. The amphoteric surfactants may be alkyl amidopropyl betaines, alkyl dimethyl betaines, alkyl amphoacetates or -diacetates. The alkyl groups of these compounds, which may be partially or fully replaced by alkenyl groups, may contain 8 to 22 carbon atoms and may be linear or branched. The polyalkylene glycol groups may contain 1 to 20 ethoxy and/or propoxy units.

Depending on the intended use, the aqueous surfactant solution according to the invention may comprise, in addition to said surfactants and auxiliaries additional additives which are customary and specific in each case, for example additives for dissolution time control or additives for performance improvement.

Suitable additives for dissolution time control are ethanol, isopropanol; butylglycol; di-butylglycol; mono-, di-, tri- or tetra-C₂-C₅ alkylene glycol such as ethylene glycol or propylene glycol, in particular mono-, di-, tri-, or tetra-propylene glycol;
polyethylene glycols (PEGs) having molecular weights of at least 400, in particular PEGs of molecular weight ranging from 6,000 to 35,000; glycerol; sugar; and mixtures thereof.

In one preferred embodiment of the present invention the aqueous surfactant solution comprise besides water one or more additional solvent components these being contained in the inventive aqueous surfactant solution preferably in an amount of 20 % by weight or less, more preferably in an amount of 10 % by weight or less and even more preferably in an amount of 8 % by weight or less, the amounts of the one or more solvent components being based on the total weight of the aqueous surfactant solution according to the invention. Among the inventive aqueous surfactant solutions comprising one or more solvent components those comprising propylene glycol and/or glycerol, preferably propylene glycol and glycerol, are preferred.

In a particularly preferred embodiment of the invention the aqueous surfactant solutions consist of components a), b) and c), wherein component c) comprises propylene glycol and glycerol.

In another preferred embodiment the inventive aqueous surfactant solutions comprise no or up to 3 % by weight, referring to the total amount of the composition, of methanol and/or ethanol and very preferred 10 % by weight or less of propylene glycol and/or of glycerol and no or up to 3 % by weight of methanol and/or ethanol.

Suitable additives for performance improvement include soil release polymers; dye fixatives; dye transfer inhibitors; bleach systems; biocides or antimicrobial components; or builders.

Suitable soil release polymers are copolymers of acrylic acid and maleic acid (Sokalan^{®} CP - BASF), homo- and copolymers of vinylpyrrolidone, vinylimidazole and nonionic monomers (Sokalan^{®} HP - BASF), homopolymers of acrylic acid (Sokalan^{®} PA - BASF), polyethylene terephthalate (PET) and polyoxyethylene terephthalate (POET) (Texcare^{®} products - Clariant). Further suitable soil release polymers are, for example, cellulose ethers or polycondensates based on dibasic carboxylic acids and reactants which possess two or more hydroxyl groups. The dibasic carboxylic acid used is typically terephthalic acid. These soil release polymers may be nonionic or anionic.

The dye fixatives which can be incorporated into the aqueous surfactant solutions according to the invention are nonionic or cationic and are described below: Polycondensates which can be used as dye fixatives are obtained by the reaction of cyanamides with aldehydes and ammonium salts and/or monoamines (e.g. dye fixative DF3), by the reaction of monoamines and/or polyamines with epichlorohydrin (e.g. dye fixatives DF2 and DF4) or by the reaction of polyamines with cyanamides and amidosulfuric acid (e.g. dye fixative DF1). The monoamines used may be primary, secondary and tertiary amines. They may be aliphatic amines, for example dialkylamines, especially dimethylamine, alicyclic amines, for example cyclohexylamine, and aromatic amines, for example aniline. However, the amines used may also simultaneously have aliphatic, alicyclic and aromatic substituents. In addition, it is also possible to use heterocyclic compounds, for example pyridine.

The term "polyamines" here includes, for example diamines, triamines, tetraamines, etc, and also the analogous N-alkylpolyamines and N,N-dialkylpolyamines. Examples thereof are ethylenediamine, propylenediamine, butylenediamine, pentylenediamine, hexylenediamine, diethylenetriamine, triethylenetetraamine and higher polyamines. Particularly preferred polyamines are ethylenediamine, diethylenetriamine and dimethylaminopropylamine. The ammonium salts are salts of ammonia, especially ammonium chloride or the abovementioned amines or polyamines with different inorganic or organic acids, or else quaternary ammonium salts.

The cyanamides may be cyanamide or dicyandiamide.

Aldehydes which can be used for the synthesis of the dye fixatives are, for example, aliphatic aldehydes, for example formaldehyde, acetaldehyde, propionaldehyde, butyraldehyde; dialdehydes, for example glyoxal; unsaturated aldehydes, for example acrolein, crotonaldehyde and aromatic aldehydes, for example benzaldehyde. Particular preference is given to the aliphatic aldehydes, especially formaldehyde.

The dye fixatives used may also be homo- and copolymers based on diallyldimethylammonium chloride (DADMAC) (e.g. dye fixatives DF5, DF6 and DF7).

Copolymers based on DADMAC contain, as further components, other vinylic monomers, for example vinylimidazole, vinylpyrrolidone, vinyl alcohol, vinyl acetate, (meth)acrylic acid/ester, acrylamide, styrene, styrenesulfonic acid, acrylamidomethylpropanesulfonic acid, etc. Homopolymers based on DADMAC are obtainable under the trade name Genamin^{®} PDAC (from Clariant).

Bleach systems such as inorganic peroxygen compounds, especially hydrogen peroxide and solid peroxygen compounds which dissolve releasing hydrogen peroxide in water, such as sodium perborate and sodium carbonate perhydrate and mixtures thereof; and bleach activators, such as those from the substance classes of the N- or O-acyl compounds, for example polyacylated alkylenediamines, especially tetraacetylethylene-diamine and tetraacetylglycoluril, N-acylated hydantoins, hydrazides, triazoles, hydrotriazines, urazoles, diketopiperazines, sulfurylamides and cyanurates, and also carboxylic anhydrides, especially phthalic anhydride and substituted maleic anhydrides, carboxylic esters, especially sodium acetoxybenzenesulfonate, sodium benzoyloxybenzenesulfonate (BOBS), sodium nonanoyloxybenzenesulfonate (NOBS), sodium isononanoyloxybenzenesulfonate (ISONOBS), and acylated sugar derivatives such as pentaacetylglucose, and mixtures thereof.

Biocides or antimicrobial components are any known ingredient having the ability of reducing or eliminating by killing or removing the micro-organisms existing on a surface. Biocides or antimicrobial components useful herein include alcohols, aldehydes, formaldehyde releasing compounds, phenolics, acid esters carbamates, amides, dibenzamidines, pyridine derivatives and related compounds, azoles, heterocyclic N,S compounds, N haloalkylthio compounds, compounds with activated halogen atoms, surface active agents, organometallic compounds, thiocyanates, biphenyl, triazine, oxidizing agents and mixtures thereof.

Suitable builders are organic and inorganic builders and are neutral or, in particular, alkaline salts which are able to precipitate out calcium ions or bind calcium ions to form a complex. Suitable and particularly ecologically acceptable builder substances, such as finely crystalline, synthetic hydrous zeolites preferably the type NaA, which have a calcium-binding capacity in the range from 100 to 200 mg of CaO/g, are used in preference. Zeolite and phyllosilicates can be present in the composition in an amount up to 20 % by weight. Organic builders which can be used are, for example, the group consisting of amino carboxylic acid, organo aminophosphonic acid compounds, and mixtures thereof. Those components, which are acidic in nature, having for example phosphonic acid or carboxylic acid functionalities, may be present either in their acid form or as a complex/salt with a suitable counter cation such as an alkali or alkaline metal ion, ammonium, or substituted ammonium ion, or any mixtures thereof. Suitable components for use herein include the amino carboxylic acids such as ethylenediamine-N,N'-disuccinic acid (EDDS), ethylenediamine tetraacetic acid (EDTA), N-hydroxyethylenediamine triacetic acid, nitrilotriacetic acid (NTA), ethylene diamine tetrapropionic acid, ethylenediamine-N,N '-diglutamic acid, 2-hydroxypropylenediamine-N,N'-disuccinic acid, triethylenetetraamine hexacetic acid, diethylenetriamine pentaacetic acid (DETPA), trans 1,2 diaminocyclohexane-N,N,N',N'-tetraacetic acid or ethanoldiglycine. Other suitable components for use herein include the organo aminophosphonic acids such as ethylenediamine tetrakis (methylenephosphonic acid), diethylene triamine-N,N,N',N",N"-pentakis (methylene phosphonic acid) (DETMP), 1-hydroxyethane 1,1-diphosphonic acid (HEDP) or hydroxyethane dimethylenephosphonic acid. It is also possible to use polymeric carboxylates and salts thereof. These include, for example, the salts of homopolymeric or copolymeric polyacrylates, polymethylacrylates and in particular, copolymers of acrylic acid with maleic acid, and also polyvinylpyrrolidone and urethanes. The relative molecular mass of the homopolymers is generally between 1000 and 100,000, that of the copolymers is between 2000 and 200,000, preferably 50,000 to 120,000, based on the free acid, in particular water-soluble polyacrylates which have been crosslinked, for example, with approximately 1 % of a sugar polyallyl ether and which have a relative molecular mass above one million are also suitable. Examples thereof are the polymers obtainable under the name Carbopol^{®} 940 and 941.

In general, additionally present in small use concentrations may be additive constituents which can be summarized under the term "washing assistants" and which thus include different active substance groups such as for example enzymes, especially proteases, lipases, cellulases, amylases and mannanases; enzyme stabilizers; preservatives; foam enhancers; foam inhibitors such as silicone oils or paraffins; corrosion inhibitors; optical brighteners; UV absorbers; alkalis; hydrotropic compounds; antioxidants; solvents; salts or extenders; dispersants; graying inhibitors; softeners; antistats; dyes, fragrances and perfumes.

Suitable enzymes are those from the class of proteases, lipases, amylases and their mixture. Their proportion can be from 0.1 to 1 % by weight. The enzymes can be adsorbed to carrier substances and/or embedded into coating substances. Suitable preservatives are, for example, phenoxy ethanol, formaldehyde solution, pentanediol or sorbic acid.

In a further preferred embodiment of the invention the additives c) comprise one or more solvents, preferably lower alkyl ethers of ethylene glycol, propylene glycol, polyethylene glycol and polypropylene glycol. "Lower alkyl" preferably means alkyl groups with 1 to 4 carbon atoms.

Suitable salts or extenders are, for example, sodium chloride, sodium sulfate, sodium carbonate, sodium silicate (water glass), magnesium chloride, or magnesium sulfate.

The aesthetics of the aqueous surfactant solutions according to the invention may be further enhanced by the addition of a dye, pigment, colorant or a pearlizing agent. Typical examples of dyes are methylene blue (Basic Blue 9), Direct Yellow 50, Solvent Red 17, Reactive Green 12 (Phthalocyanine, Clariant).

In a more preferred embodiment of the present invention, the aqueous surfactant solutions comprise one or more substances selected from the group consisting of preservatives, antimicrobial components, enzymes, dye transfer inhibitors, soil release polymers, cationic surfactants, anionic surfactants, amphoteric surfactants, propylene glycol, glycerol, dyes, pigments and fragrances.

According to a further preferred embodiment of the present invention the aqueous surfactant solution consists of the components a), b) and c).

Aqueous surfactant solutions according to the invention are obtained by mixing components a), b) and c) by usual methods known to a skilled person.

The aqueous surfactant solutions according to the invention are advantageously suited for cleaning, scenting, conditioning or disinfection purposes, preferably in home or personal care applications.

### Fourth Aspect

d) Therefore, a further subject matter of the present invention is the use of the one or more, preferably one, compound(s) of the formula (I) according to the invention or of the blend of N-acylglucamide compounds of the formulas (Ia) and (Ib) according to the invention for cleaning, scenting, conditioning or disinfection purposes, preferably in home or personal care applications.

Furthermore the the one or more, preferably one, compound(s) of the formula (I) according to the invention or of the blend of N-acylglucamide compounds of the formulas (Ia) and (Ib) according to the invention can be used as a corrosion inhibitor (e.g. for metal work fluid formulation) or as a lubricant.

### Fifth Aspect

A fifth aspect relates to use of the aqueous surfactant solution according to the invention for the afore-mentioned purposes.

For use the aqueous surfactant solutions according to the invention are preferably applied to surfaces made of ceramics, plastic or stainless steel, or are applied in laundry machines or dishwashers.

### EXAMPLES

### EXPERIMENTAL

The examples which follow are intended to illustrate the subject matter of the invention without restricting it thereto.

The following examples further illustrate various properties of the compounds of the formula (I) according to the invention, and of the blend of N-acylglucamide compounds of the formulas (Ia) and (Ib) according to the invention.

### Materials

The following raw materials were used:
Methyl-3-hydroxytetradecanoate, partially unsaturated, containing mainly (more than 90 wt.-%) C14 saturated and mono unsaturated beta-hydroxy methyl ester.
Methyl-16-hydroxyhexadecanoate, partially unsaturated, containing mainly (more than 90 wt.-%) C16 saturated and mono unsaturated omega-hydroxy methyl ester.

Methyl-3-hydroxytetradecanoate, completely saturated, containing mainly (more than 90 wt.-%) C14 saturated beta-hydroxy methyl ester.

Methyl-16-hydroxyhexadecanoate, completely saturated, containing mainly (more than 90 wt.-%) C16 saturated omega-hydroxy methyl ester.

N-methylglucamine and sodium methoxide were purchased from Sigma-Aldrich.

### General procedure for the preparation of N-acylglucamides

The amounts of N-methylglucamine, fatty acid methyl ester, sodium methoxide as catalyst and optionally solvent given in the reaction protocols below were charged into a five-neck flask equipped with distillation condenser, overhead stirrer, internal thermometer and nitrogen inlet tube. The temperature of the mixture was increased to 130°C while gently stirring. As the temperature approached 130°C, the mixture slowly melted to a tan liquid. Heating and stirring were continued with continuous removal of methanol from the reaction mixture.

The progress of the reaction was monitored by potentiometric amine number titration of aliquots of the reaction mixture with perchloric acid. Amine number is abbreviated as AN. It was determined by potentiometric titration of the sample with perchloric acid after dilution of the sample with acetic acid. When titration showed AN ≤ 1 mmol/g, the formation of the N-acylglucamides was considered to be completed. The reaction progress was followed by means of 1H-NMR spectroscopy. When the three hydrogen signals of the methyl esters at δ = 3.67 ppm in the 1H-NMR spectrum were no longer visible the reaction was stopped. After the completion of the reaction, the residual methanol was removed from the reaction mixture to yield N-acylglucamides. The N-acylglucamides were characterized by 1H-NMR and infrared (IR) spectroscopy.

**Table 1: Chemical Components Used in Synthesis**

| Example | starting material/ g (mol) | N-methylglucamine g (mol) | wt.% sodium methoxide | 20 wt.% Solvent |
|---|---|---|---|---|
| 1 | Methyl-3-hydroxytetradecanoate, partially unsaturated/ 20.0 (76.40) | 14.91 (76.40) | 5 | none |
| 2 | Methyl-3-hydroxytetradecanoate, completely saturated/ 33.78 (131.37) | 25.64 (131.37) | 5 | none |
| 3 | Methyl-16-hydroxyhexadecanoate, partially unsaturated/ 34.76 (126.46) | 24.69 (126.46) | 5 | none |
| 4 | Methyl-16-hydroxyhexadecanoate, completely saturated/ 34.76 (126.46) | 24.69 (126.46) | 5 | none |
| 5 | Methyl-16-hydroxyhexadecanoate, partially unsaturated/ 34.76 (126.46) | 24.69 (126.46) | 5 | methanol |
| 6 | Methyl-3-hydroxytetradecanoate, completely saturated/ 33.78 (131.37) | 25.64 (131.37) | 5 | methanol |

**Table 2: Synthetic and analytical results**

| Example | | 1 | 2 | 3 | 4 | 5 | 3 |
|---|---|---|---|---|---|---|---|
| Yield % | | 90 | 93 | 91 | 95 | 36 | 35 |
| 1H-NMR (δ ppm) | N-CH₃ | 2.42 | 2.43 | 2.30 | 2.34 | 2.30 | 2.43 |
| | CH₃ | 0.89 | 0.90 | 0.88 | 0.91 | 0.88 | 0.90 |
| | CH=CH | 5.34 | none | 5.34 | none | 5.34 | none |
| IR (cm⁻¹) | C=O (stretching) | 1610.77 | 1605.25 | 1617.71 | 1617.56 | 1617.71 | 1605.25 |
| | O-H (stretching) | 3333.76 | 3327.37 | 3340.40 | 3327.67 | 3340.40 | 3327.37 |
| | C-H (stretching) | 2852.65 | 2920.16 | 2920.66 | 2915.16 | 2920.66 | 2920.16 |
| | C-N (bending) | 1077.84 | 1077.95 | 1052.98 | 1073.65 | 1052.98 | 1077.95 |

The following examples are offered to illustrate various properties of the surface active agents of the present invention. Those skilled in the art will understand that they are not to be construed as limiting the scope and spirit of the invention.

### Surface tension and Critical micelle concentration (CMC)

Surface tension and Critical micelle concentration (CMC) were determined by a Kruess Tensiometer K 100 (Ring) apparatus at a concentration of 10 g/L of sample in water, measured at room temperature (25 °C).

**Table 3: Surface tension and Critical micelle concentration (CMC)**

| Example | Surface tension (mN/m) | Critical micelle concentration (g/L) |
|---|---|---|
| 1 (inventive) | 29.8 | 0.049 |
| 2 (inventive) | | |
| 3 (inventive) | 31.0 | 0.011 |
| 4 (inventive) | | |
| 5* (comparative)** | 27.6 | 0.060 |
| 6* (comparative)** | 37.0 | 0.0035 |
| *Example 5 = N-methyl-N-C₁₂/C₁₄-acylglucamide; | | |
| *Example 6 = N-methyl-N-C₁₆/C₁₈-acylglucamide | | |
| **Comparative example 5 and 6 are prepared as described in EP 0 550 637. | | |

### Foam (collapse) test

20 mL of 1 wt.% solution of each substance were placed into a graduated 100 mL measurement cylinder, which was sealed with a stopper. Foam was generated by 20 shakes. The foam height was determined as mL foam above the aqueous layer directly after shaking, after 2, 3 and 5 min.

**Table 4: Foam Height (mL)**

| Product of Example | Initial | 2 min | 3 min | 5 min |
|---|---|---|---|---|
| 1 | 80 | 62 | 34 | 20 |
| 2 | 80 | 60 | 33 | 19 |
| 3 | 91 | 70 | 51 | 35 |
| 4 | 89 | 67 | 50 | 33 |

### Wetting test

Cotton swatches (ISO 8022, obtained from wfk-Testgewebe GmbH) were placed in 600 mL beaker, filled with the corresponding 0.1 wt.% surfactant solution in water. The time between placing the swatches into the solution and sinking to the bottom was measured.

**Table 5: Wetting Test**

| Product of Example | Wetting time (s) | Performance |
|---|---|---|
| 1 | 30 | good |
| 2 | 60 | good |
| 3 | 140 | Medium good |
| 4 | >300 | poor |

The experimental data demonstrate that the N-acylglucamides of the present invention are effective surfactants. The N-acylglucamides provide an excellent performance as surfactant, which is shown by an excellent foamability and stability of the formed foam which is stable for several minutes (cp. Table 4). Further, the surface tension of the blends of examples 1 and 3 is good. Furthermore, example 1 have an improved CMC value compared to the comparative example 5. (cp. Table 3).

## Claims

1. N-acylglucamide compound of the formula (I) 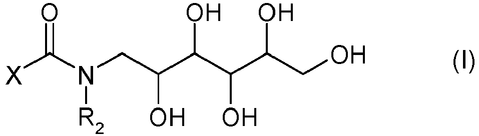 where
R₂ is a linear or branched alkyl group or an aryl group;
X is a moiety R₁, which is a linear or branched alkyl group comprising 8 to 18, carbon atoms, which is substituted by one hydroxy group preferably in beta- or omega-position; or
X is a moiety R₁, which is a linear or branched, mono- or polyunsaturated alkenyl group comprising 8 to 18 carbon atoms, which is substituted by one hydroxy group preferably in beta- or omega-position.

2. N-acylglucamide compound according to claim 1 where
R₂ is a linear alkyl group comprising 1 to 6 carbon atoms, preferably a methyl group;
X is a moiety R₁, which is a linear alkyl group comprising 13 to 17, preferably 13 to 15, carbon atoms, which is substituted by one hydroxy group in beta- or omega-position; or
X is a moiety R_{1'}, which is a linear, monounsaturated alkenyl group comprising 13 to 17, preferably 13 to 15, carbon atoms, which is substituted by one hydroxy group in beta- or omega-position.

3. N-acylglucamide compound according to claim 1 or 2 where
R₂ is a methyl group;
X is a moiety R₁, which is a linear alkyl group with 13 carbon atoms substituted by a hydroxy group in beta-position; or
X is a moiety R_{1'}, which is a linear, monounsaturated alkenyl group with 13 carbon atoms substituted by a hydroxy group in beta-position.

4. N-acylglucamide compound according to claim 1 or 2 where
R₂ is a methyl group;
X is a moiety R₁, which is a linear alkyl group with 15 carbon atoms substituted by a hydroxy group in omega-position; or
X is a moiety R_{1'}, which is a linear, monounsaturated alkenyl group with 15 carbon atoms substituted by a hydroxy group in omega-position.

5. Blend consisting of at least one N-acylglucamide compound of the formula (Ia) 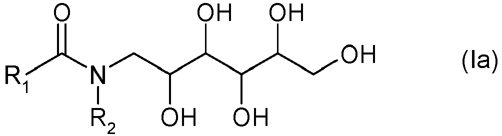 and at least one N-acylglucamide compound of the formula (Ib) 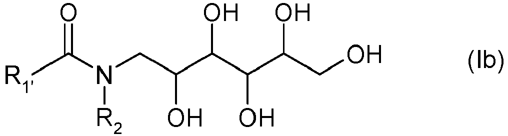 wherein R₂, R₁ and R_{1'} are as defined in any of claims 1 to 4.

6. Blend according to claim 5, where the compounds of the formulas (Ia) and (Ib) are identical, except of the extent of unsaturation of the moieties R₁ and R_{1'}.

7. Blend according to claim 5 or 6, where the amount of the compound of the formula (Ib) - based on the blend of (Ia) and (Ib) - is at least 35 wt.-%, preferably at least 40 wt.-%, more preferably at least 65 wt.-%, most preferably at least 70 wt.-%.

8. A process for the preparation of compounds of the formula (I) according to any of claims 1 to 4 or the blend according to any of claims 5 to 7, wherein the process comprises:
i) Providing fatty acids or fatty acid derivatives of the formula XCOR³, wherein X is as described in formula (I) above and R³ is hydroxy, a linear or branched C₁-C₄-alkoxy group or halogen;
ii) Amidation of the fatty acid or fatty acid derivative of the formula XCOR³, with an N-alkyl- or aryl-glucamine, preferably N-alkyl-glucamine, more preferably N-methyl-glucamine, in a polar protic solvent in presence of a base catalyst.

9. The process according to claim 8 wherein the fatty acids or fatty acid derivatives of the formula XCOR³, in particular fatty acid esters of the formula XCOO(C₁-C₄-alkyl), provided in step i) are obtained by living organisms from simple carbon renewable raw materials, preferably carbohydrates, in a host cell, which is optionally genetically engineered, that contains one or more naturally-occurring mutations.

10. An aqueous surfactant solution comprising one or more compound(s) of the formula (I) according to any of claims 1 to 4, or the blend according to any of claims 5 to 7.

11. The aqueous surfactant solution of claim 10 comprising:
a) 0.1 to 10 wt.-% of one or more compound(s) of the formula (I) according to any of claims 1 to 4, or of the blend according to any of claims 5 to 7,
b) 50 to 99.9 wt.-% of water,
c) 0 to 49.9 wt.-% of one or more further additives,
wherein a), b) and c) sum up to 100 wt.-%.

12. The aqueous surfactant solution according to claim 10 or 11 comprising:
0.1 to 5 wt.-% of one or more compound(s) of the formula (I) according to any of claims 1 to 4, or of the blend according to any of claims 5 to 7,
75 to 99.9 wt.-% of water,
0 to 20 wt.-% of one or more additives,
wherein a), b) and c) sum up to 100 wt.-%.

13. Use of the compounds of the formula (I) according to any of claims 1 to 4, or of the blend according to any of claims 5 to 7 for cleaning, scenting, conditioning or disinfection purposes, preferably in home or personal care applications, or as a corrosion inhibitor or lubricant.

14. Use of the aqueous surfactant solution according to any of claims 10 to 11 for cleaning, scenting, conditioning or disinfection purposes, preferably in home or personal care applications, or as a corrosion inhibitor or lubricant.

## Patentansprüche

1. N-Acylglucamid-Verbindung der Formel (I) 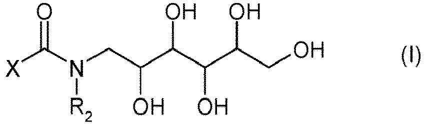 wobei
R₂ für eine lineare oder verzweigte Alkylgruppe oder eine Arylgruppe steht;
X für eine Gruppierung R₁ steht, bei der es sich um eine lineare oder verzweigte Alkylgruppe mit 8 bis 18 Kohlenstoffatomen, die durch eine Hydroxylgruppe, vorzugsweise in beta- oder omega-Position, substituiert ist, handelt; oder
X für eine Gruppierung R_{1'} steht, bei der es sich um eine lineare oder verzweigte, ein- oder mehrfach ungesättigte Alkenylgruppe mit 8 bis 18 Kohlenstoffatomen, die durch eine Hydroxylgruppe, vorzugsweise in beta- oder omega-Position, substituiert ist, handelt.

2. N-Acylglucamid-Verbindung nach Anspruch 1, wobei
R₂ für eine lineare Alkylgruppe mit 1 bis 6 Kohlenstoffatomen, vorzugsweise eine Methylgruppe, steht;
X für eine Gruppierung R₁ steht, bei der es sich um eine lineare Alkylgruppe mit 13 bis 17, vorzugsweise 13 bis 15, Kohlenstoffatomen, die in beta- oder omega-Position durch eine Hydroxylgruppe substituiert ist, handelt; oder
X für eine Gruppierung R_{1'} steht, bei der es sich um eine lineare, einfach ungesättigte Alkenylgruppe mit 13 bis 17, vorzugsweise 13 bis 15, Kohlenstoffatomen, die in beta- oder omega-Position durch eine Hydroxylgruppe substituiert ist, handelt.

3. N-Acylglucamid-Verbindung nach Anspruch 1 oder 2, wobei
R₂ für eine Methylgruppe steht;
X für eine Gruppierung R₁ steht, bei der es sich um eine lineare Alkylgruppe mit 13 Kohlenstoffatomen, die in beta-Position durch eine Hydroxylgruppe substituiert ist, handelt; oder
X für eine Gruppierung R_{1'} steht, bei der es sich um eine lineare, einfach ungesättigte Alkenylgruppe mit 13 Kohlenstoffatomen, die in beta-Position durch eine Hydroxylgruppe substituiert ist, handelt.

4. N-Acylglucamid-Verbindung nach Anspruch 1 oder 2, wobei
R₂ für eine Methylgruppe steht;
X für eine Gruppierung R₁ steht, bei der es sich um eine lineare Alkylgruppe mit 15 Kohlenstoffatomen, die in omega-Position durch eine Hydroxylgruppe substituiert ist, handelt; oder
X für eine Gruppierung R_{1'} steht, bei der es sich um eine lineare, einfach ungesättigte Alkenylgruppe mit 15 Kohlenstoffatomen, die in omega-Position durch eine Hydroxylgruppe substituiert ist, handelt.

5. Mischung, bestehend aus mindestens einer N-Acylglucamid-Verbindung der Formel (Ia) 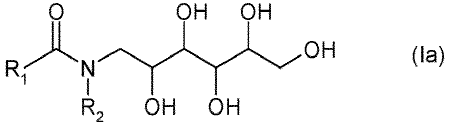 und mindestens einer N-Acylglucamid-Verbindung der Formel (Ib) 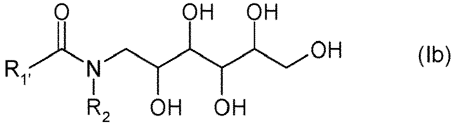 wobei R₂, R₁ und R_{1'} wie in einem der Ansprüche 1 bis 4 definiert sind.

6. Mischung nach Anspruch 5, wobei die Verbindungen der Formeln (Ia) und (Ib) bis auf das Ausmaß der Ungesättigtheit der Gruppierungen R₁ und R₁, identisch sind.

7. Mischung nach Anspruch 5 oder 6, wobei die Menge der Verbindung der Formel (Ib) - bezogen auf die Mischung von (Ia) und (Ib) - mindestens 35 Gew.-%, vorzugsweise mindestens 40 Gew.-%, weiter bevorzugt mindestens 65 Gew.-%, ganz besonders bevorzugt mindestens 70 Gew.-%, beträgt.

8. Verfahren zur Herstellung der Verbindungen der Formel (I) nach einem der Ansprüche 1 bis 4 oder der Mischung nach einem der Ansprüche 5 bis 7, wobei das Verfahren Folgendes umfasst:
i) Bereitstellen von Fettsäuren oder Fettsäurederivaten der Formel XCOR³, wobei X wie in obiger Formel (I) beschrieben ist und R³ für Hydroxy, eine lineare oder verzweigte C₁-C₄-Alkoxygruppe oder Halogen steht;
ii) Amidierung der Fettsäure oder des Fettsäurederivats der Formel XCOR³ mit einem N-Alkyl- oder Arylglucamin, vorzugsweise N-Alkylglucamin, weiter bevorzugt N-Methylglucamin, in einem polaren protischen Lösungsmittel in Gegenwart eines Basenkatalysators.

9. Verfahren nach Anspruch 8, wobei die in Schritt i) bereitgestellten Fettsäuren oder Fettsäurederivate der Formel XCOR³, insbesondere Fettsäureester der Formel XCOO(C₁-C₄-Alkyl), durch lebende Organismen aus einfachen nachwachsenden Kohlenstoffrohstoffen, vorzugsweise Kohlenhydraten, in einer gegebenenfalls gentechnisch veränderten Wirtszelle, die eine oder mehrere natürlich vorkommende Mutationen enthält, erhalten werden.

10. Wässrige Tensidlösung, umfassend eine oder mehrere Verbindungen der Formel (I) nach einem der Ansprüche 1 bis 4 oder die Mischung nach einem der Ansprüche 5 bis 7.

11. Wässrige Tensidlösung nach Anspruch 10, umfassend:
a) 0,1 bis 10 Gew.-% einer oder mehrerer Verbindungen der Formel (I) nach einem der Ansprüche 1 bis 4 oder der Mischung nach einem der Ansprüche 5 bis 7,
b) 50 bis 99,9 Gew.-% Wasser,
c) 0 bis 49,9 Gew.-% eines oder mehrerer weiterer Additive,
wobei sich a), b) und c) zu 100 Gew.-% summieren.

12. Wässrige Tensidlösung nach Anspruch 10 oder 11, umfassend:
0,1 bis 5 Gew.-% einer oder mehrerer Verbindungen der Formel (I) nach einem der Ansprüche 1 bis 4 oder der Mischung nach einem der Ansprüche 5 bis 7,
75 bis 99,9 Gew.-% Wasser,
0 bis 20 Gew.-% eines oder mehrerer weiterer Additive, wobei sich a), b) und c) zu 100 Gew.-% summieren.

13. Verwendung der Verbindungen der Formel (I) nach einem der Ansprüche 1 bis 4 oder der Mischung nach einem der Ansprüche 5 bis 7 für Reinigungs-, Beduftungs-, Konditionierungs- oder Desinfektionszwecke, vorzugsweise bei Heimpflege- oder Körperpflegeanwendungen, oder als Korrosionsinhibitor oder Schmiermittel.

14. Verwendung der wässrigen Tensidlösung nach einem der Ansprüche 10 bis 11 für Reinigungs-, Beduftungs-, Konditionierungs- oder Desinfektionszwecke, vorzugsweise bei Heimpflege- oder Körperpflegeanwendungen, oder als Korrosionsinhibitor oder Schmiermittel.

## Revendications

1. Composé de type N-acylglucamide de la formule (I) 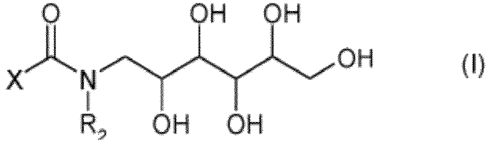 où
R₂ est un groupe alkyle linéaire ou ramifié ou un groupe aryle ;
X est un groupement R₁, qui est un groupe alkyle linéaire ou ramifié comprenant 8 à 18 atomes de carbone, qui est substitué par un groupe hydroxy préférablement en position bêta ou oméga ; ou
X est un groupement R_{1'}, qui est un groupe alcényle linéaire ou ramifié, monoinsaturé ou polyinsaturé comprenant 8 à 18 atomes de carbone, qui est substitué par un groupe hydroxy préférablement en position bêta ou oméga.

2. Composé de type N-acylglucamide selon la revendication 1 où
R₂ est un groupe alkyle linéaire comprenant 1 à 6 atomes de carbone, préférablement un groupe méthyle ;
X est un groupement R₁, qui est un groupe alkyle linéaire comprenant 13 à 17, préférablement 13 à 15, atomes de carbone, qui est substitué par un groupe hydroxy en position bêta ou oméga ; ou
X est un groupement R_{1'}, qui est un groupe alcényle linéaire, monoinsaturé comprenant 13 à 17, préférablement 13 à 15, atomes de carbone, qui est substitué par un groupe hydroxy en position bêta ou oméga.

3. Composé de type N-acylglucamide selon la revendication 1 ou 2 où
R₂ est un groupe méthyle ;
X est un groupement R₁, qui est un groupe alkyle linéaire comprenant 13 atomes de carbone substitué par un groupe hydroxy en position bêta ; ou
X est un groupement R_{1'}, qui est un groupe alcényle linéaire, monoinsaturé comprenant 13 atomes de carbone substitué par un groupe hydroxy en position bêta.

4. Composé de type N-acylglucamide selon la revendication 1 ou 2 où
R₂ est un groupe méthyle ;
X est un groupement R₁, qui est un groupe alkyle linéaire comprenant 15 atomes de carbone substitué par un groupe hydroxy en position oméga ; ou
X est un groupement R_{1'}, qui est un groupe alcényle linéaire, monoinsaturé comprenant 15 atomes de carbone substitué par un groupe hydroxy en position oméga.

5. Mélange constitué d'au moins un composé de type N-acylglucamide de la formule (Ia) 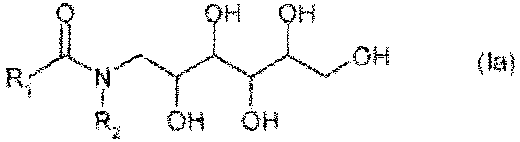 et d'au moins un composé de type N-acylglucamide de la formule (Ib) 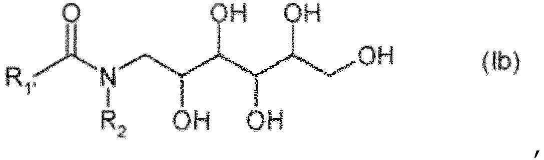 R₂, R₁ et R_{1'} étant tels que définis dans l'une quelconque des revendications 1 à 4.

6. Mélange selon la revendication 5, où les composés des formules (Ia) et (Ib) sont identiques, à l'exception de l'étendue d'insaturation des groupements R₁ et R_{1'}.

7. Mélange selon la revendication 5 ou 6, où la quantité du composé de la formule (Ib) - sur la base du mélange de (Ia) et (Ib) - est d'au moins 35 % en poids, préférablement d'au moins 40 % en poids, plus préférablement d'au moins 65 % en poids, le plus préférablement d'au moins 70 % en poids.

8. Procédé pour la préparation de composés de la formule (I) selon l'une quelconque des revendications 1 à 4 ou du mélange selon l'une quelconque des revendications 5 à 7, le procédé comprenant :
i) la fourniture d'acides gras ou de dérivés d'acides gras de la formule XCOR³, X étant tel que décrit dans la formule (I) ci-dessus et R³ étant hydroxy, un groupe C₁₋₄-alcoxy linéaire ou ramifié ou halogène ;
ii) l'amidation de l'acide gras ou du dérivé d'acide gras de la formule XCOR³, avec une N-alkyl-glucamine ou une N-aryl-glucamine, préférablement une N-alkyl-glucamine, plus préférablement la N-méthyl-glucamine, dans un solvant protique polaire en présence d'un catalyseur de type base.

9. Procédé selon la revendication 8, les acides gras ou dérivés d'acides gras de la formule XCOR³, en particulier les esters d'acides gras de la formule XCOO(C₁₋₄-alkyle) , fournis dans l'étape i) étant obtenus par des organismes vivants à partir de matières premières renouvelables carbonées simples, préférablement des glucides, dans une cellule hôte, qui est éventuellement génétiquement modifiée, qui contient une ou plusieurs mutations d'origine naturelle.

10. Solution aqueuse de tensioactif comprenant un ou plusieurs composé(s) de la formule (I) selon l'une quelconque des revendications 1 à 4, ou le mélange selon l'une quelconque des revendications 5 à 7.

11. Solution aqueuse de tensioactif selon la revendication 10 comprenant :
a) 0,1 à 10 % en poids d'un ou plusieurs composé(s) de la formule (I) selon l'une quelconque des revendications 1 à 4, ou du mélange selon l'une quelconque des revendications 5 à 7,
b) 50 à 99,9 % en poids d'eau,
c) 0 à 49,9 % en poids d'un ou plusieurs autres additifs,
a), b) et c) totalisant 100 % en poids.

12. Solution aqueuse de tensioactif selon la revendication 10 ou 11 comprenant :
0,1 à 5 % en poids d'un ou plusieurs composé(s) de la formule (I) selon l'une quelconque des revendications 1 à 4, ou du mélange selon l'une quelconque des revendications 5 à 7,
75 à 99,9 % en poids d'eau,
0 à 20 % en poids d'un ou plusieurs additifs,
a), b) et c) totalisant 100 % en poids.

13. Utilisation des composés de la formule (I) selon l'une quelconque des revendications 1 à 4, ou du mélange selon l'une quelconque des revendications 5 à 7 à des fins de nettoyage, de parfumage, de conditionnement ou de désinfection, préférablement dans des applications d'entretien ménager ou personnel, ou en tant qu'inhibiteur de corrosion ou lubrifiant.

14. Utilisation de la solution aqueuse de tensioactif selon l'une quelconque des revendications 10 à 11 à des fins de nettoyage, de parfumage, de conditionnement ou de désinfection, préférablement dans des applications d'entretien ménager ou personnel, ou en tant qu'inhibiteur de corrosion ou lubrifiant.
